(19) Europäisches Patentamt
European Patent Office
Office européen des brevets

(11) Veröffentlichungsnummer: **0 128 382**
**B1**

(12) # EUROPÄISCHE PATENTSCHRIFT

(45) Veröffentlichungstag der Patentschrift: **27.09.89**

(51) Int. Cl.4: **C 07 C 119/045**, C 07 C 118/00, C 07 C 87/38

(21) Anmeldenummer: **84105472.9**

(22) Anmeldetag: **14.05.84**

(54) **1-Alkyl-2-isocyanatomethyl-isocyanato-cyclohexane und/oder 1-Alkyl-4-isocyanatomethyl-isocyanato-cyclohexane sowie die entsprechenden Diamine, Verfahren zu deren Herstellung und Verwendung.**

(30) Priorität: **17.05.83 DE 3317875**

(43) Veröffentlichungstag der Anmeldung: **19.12.84 Patentblatt 84/51**

(45) Bekanntmachung des Hinweises auf die Patenterteilung: **27.09.89 Patentblatt 89/39**

(84) Benannte Vertragsstaaten: **BE CH DE FR GB IT LI SE**

(56) Entgegenhaltungen:
**CH-A- 572 460**
**US-A- 2 692 275**

**BULLETIN OF THE CHEMICAL SOCIETY OF JAPAN, Band 56, Nr. 2, Februar 1983, R. SAITO et al. "Studies of the metal complexes of cyclohexane derivatives. VII. Preparation and properties of platinum(II) and cobalt(III) complexes of (1R,2S)-1-aminomethyl-2-methylcyclohexylamine"**

(73) Patentinhaber: **BASF Aktiengesellschaft, Carl-Bosch-Strasse 38, D-6700 Ludwigshafen (DE)**

(72) Erfinder: **Hahn, Erwin, Dr., Am Buechsenackerhang 31, D-6900 Heidelberg (DE)**
Erfinder: **Neumann, Peter, Dr., Franz-Schubert-Strasse 1, D-6908 Wiesloch (DE)**

**EP 0 128 382 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Aromatische Diisocyanate, wie z.B. 2,4- und 2,6-Toluylendiisocyanat, 1,5-Naphthylen-diisocyanat, 4,4'-Diphenylmethandiisocyanat und Mischungen aus 4,4'-, 2,4'- und 2,2'-Diphenylmethandiisocyanaten und Polyphenyl-polymethylenpolyisocyanaten, aliphatische Diisocyanate, wie z.B. Hexamethylendiisocyanat-1,6 und cycloaliphatische Diisocyanate, wie z.B. 3-Isocyanatomethyl-3,5,5-trimethyl-cyclohexylisocyanat, sind bekannte Handelsprodukte, die vorzugsweise zur Herstellung von Polyurethankunststoffen Verwendung finden. Die Polyisocyanate werden üblicherweise aus den entsprechenden Aminoverbindungen durch Phosgenierung und anschließende thermische Spaltung der intermediär gebildeten Carbaminsäurechloride hergestellt. Zahlreiche organische Mono- und Polyisocyanate werden beispielsweise beschrieben in Annalen der Chemie 562 (1949), Seiten 75 ff.

Aufgabe der vorliegenden Erfindung war es, cycloaliphatische Diisocyanate zu entwickeln, die Isocyanatgruppen mit einer unterschiedlichen Reaktivität gebunden enthalten.

Gegenstand der Erfindung sind cycloaliphatische Diisocyanate der Formeln

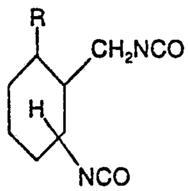
(I) oder
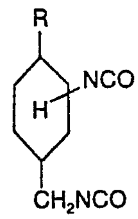
(II),

in denen R eine Alkylgruppe mit 1 bis 12 Kohlenstoffatomen bedeutet.

Die erfindungsgemäßen Diisocyanate erfüllen das genannte Erfordernis.

Bevorzugt sind Diisocyanate der Formeln

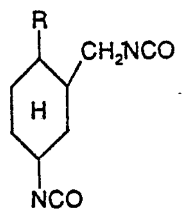
(III) oder
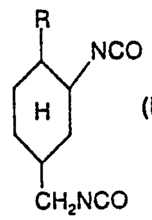
(IV),

bei denen die Isocyanato-methylgruppe in 2- oder 4-Stellung und die Isocyanatgruppe in 4- oder 2-Stellung gebunden sind.

Gegenstand der Erfindung sind außerdem Diamine der Formeln

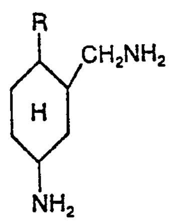
(VII) oder
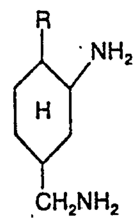
(VIII)

In den Formeln (I) bis (VIII) bedeutet R einen Alkylrest mit 1 bis 12 Kohlenstoffatomen, vorzugsweise 1 bis 4 Kohlenstoffatomen, der verzweigt sein kann, vorzugsweise jedoch linear ist. Genannt seien beispielsweise Alkylreste wie der n-Pentyl-, n-Hexyl-, n-Heptyl-, n-Octyl-, 2-Ethyl-n-hexyl-, n-Nonyl-, n-Decyl-, n-Undecyl-, n-Dodecylrest, vorzugsweise der Ethyl-, n- und iso-Propyl- und n- und sek.-Butylrest und insbesondere der Methylrest.

Als erfindungsgemäße Diisocyanate kommen im einzelnen beispielsweise in Betracht:

1-Methyl-2-isocyanatomethyl-3-isocyanato-,
1-Methyl-2-isocyanatomethyl-5-isocyanato-,
1-Methyl-2-isocyanatomethyl-6-isocyanato-,
1-Methyl-4-isocyanatomethyl-3-isocyanato-cyclohexan,
1-Ethyl-2-isocyanatomethyl-3-isocyanato-,
1-Ethyl-2-isocyanatomethyl-5-isocyanato-,
1-Ethyl-2-isocyanatomethyl-6-isocyanato-,
1-Ethyl-4-isocyanatomethyl-3-isocyanato-cyclohexan,
1-n-Propyl-2-isocyanatomethyl-3-isocyanato-,
1-n-Propyl-2-isocyanatomethyl-5-isocyanato-,
1-n-Propyl-2-isocyanatomethyl-6-isocyanato-,
1-n-Propyl-4-isocyanatomethyl-3-isocyanato-cyclohexan,
1-iso-Propyl-2-isocyanatomethyl-3-isocyanato-,
1-iso-Propyl-2-isocyanatomethyl-4-isocyanato-,
1-iso-Propyl-2-isocyanatomethyl-5-isocyanato-,
1-iso-Propyl-2-isocyanatomethyl-6-isocyanato-,
1-iso-Propyl-4-isocyanatomethyl-2-isocyanato-,
1-iso-Propyl-4-isocyanatomethyl-3-isocyanato-cyclohexan,
1-n-Butyl-2-isocyanatomethyl-3-isocyanato-,
1-n-Butyl-2-isocyanatomethyl-4-isocyanato-,

1-n-Butyl-2-isocyanatomethyl-5-isocyanato-,
1-n-Butyl-2-isocyanatomethyl-6-isocyanato-,
1-n-Butyl-4-isocyanatomethyl-2-isocyanato-,
1-n-Butyl-4-isocyanatomethyl-3-isocyanato-cyclohexan,
1-n-Pentyl-2-isocyanatomethyl-4-isocyanato-,
1-n-Pentyl-2-isocyanatomethyl-6-isocyanato-,
1-n-Pentyl-4-isocyanatomethyl-2-isocyanato-cyclohexan,
1-n-Hexyl-2-isocyanatomethyl-4-isocyanato-,
1-n-Hexyl-2-isocyanatomethyl-6-isocyanato-,
1-n-Hexyl-4-isocyanatomethyl-2-isocyanato-cyclohexan,
1-n-Heptyl-2-isocyanatomethyl-4-isocyanato-,
1-n-Heptyl-2-isocyanatomethyl-6-isocyanato-,
1-n-Heptyl-4-isocyanatomethyl-2-isocyanato-cyclohexan,
1-n-Octyl-2-isocyanatomethyl-4-isocyanato-,
1-n-Octyl-2-isocyanatomethyl-6-isocyanato-,
1-n-Octyl-4-isocyanatomethyl-2-isocyanato-cyclohexan,
1-n-Nonyl-2-isocyanatomethyl-4-isocyanato-,
1-n-Nonyl-2-isocyanatomethyl-6-isocyanato-,
1-n-Nonyl-4-isocyanatomethyl-2-isocyanato-cyclohexan,
1-n-Decyl-2-isocyanatomethyl-4-isocyanato-,
1-n-Decyl-2-isocyanatomethyl-6-isocyanato-,
1-n-Decyl-4-isocyanatomethyl-2-isocyanato-cyclohexan,
1-n-Undecyl-2-isocyanatomethyl-4-isocyanato-,
1-n-Undecyl-2-isocyanatomethyl-6-isocyanato-,
1-n-Undecyl-4-isocyanatomethyl-2-isocyanato-cyclohexan,
1-n-Dodecyl-2-isocyanatomethyl-4-isocyanato-,
1-n-Dodecyl-2-isocyanatomethyl-6-isocyanato-und
1-n-Dodecyl-4-isocyanatomethyl-2-isocyanato-cyclohexan.

Als bevorzugt genannt seien:
1-Ethyl-2-isocyanatomethyl-4-isocyanato-,
1-Ethyl-4-isocyanatomethyl-2-isocyanato-cyclohexan
1-n-Propyl-2-isocyanatomethyl-4-isocyanato-und
1-n-Propyl-4-isocyanatomethyl-2-isocyanato-cyclohexan,
sowie insbesondere
1-Methyl-2-isocyanatomethyl-4-isocyanato-,
1-Methyl-2-isocyanatomethyl-6-isocyanato-cyclohexan,
sowie deren Isomerengemische und
1-Methyl-4-isocyanatomethyl-2-isocyanato-cyclohexan.

Als Ausgangskomponente zur Herstellung der erfindungsgemäßen Diisocyanate finden die den obengenannten Diisocyanaten entsprechenden neuen Diamine Verwendung.

Besonders bewährt haben sich hierfür und daher vorzugsweise Anwendung finden:
1-Ethyl-2-aminomethyl-4-amino-,
1-Ethyl-4-aminomethyl-2-amino-cyclohexan,
1-n-Propyl-2-aminomethyl-6-amino-cyclohexan,
1-n-Propyl-2-aminomethyl-4-amino- und
1-n-Propyl-4-aminomethyl-2-amino-cyclohexan
sowie insbesondere
1-Methyl-2-aminomethyl-4-amino-,
1-Methyl-2-aminomethyl-6-amino-cyclohexan,
sowie deren Isomerengemische und
1-Methyl-4-aminomethyl-2-amino-cyclohexan.

Die erfindungsgemäßen Diisocyanate und Diamine hierfür können in Form von Isomerengemischen, Gemische gleicher Isomeren aber verschiedener Alkylreste oder als Mischungen von beiden Gemischen vorliegen.

Die Herstellung der neuen Diamine und Diisocyanate kann beispielsweise nach folgendem Verfahrensschema erfolgen:

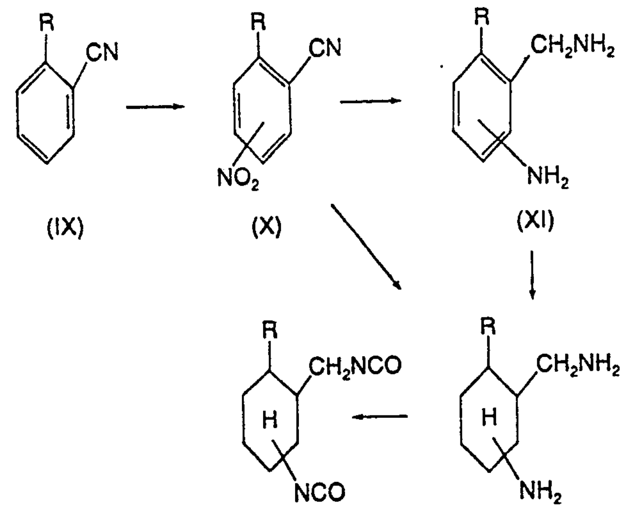

(IX) (X) (XI)

(I) (V)

Die 1-Alkyl-2-cyano-nitrobenzole (X) bzw. 1-Alkyl-4-cyano-nitrobenzole können nach an sich bekannten Verfahren durch Nitrierung der entsprechenden 1-Alkyl-2-cyano-benzole (IX) oder 1-Alkyl-4-cyano-benzole hergestellt werden, beispielsweise analog der in Berichte der Deutschen Chemischen Gesellschaft 31, Seiten 2880 ff (1898) oder in J. Amer. Chem. Soc. 99, 6721 (1977) beschriebenen Methoden oder geeigneten Varianten davon.

Eine andere, beispielhaft genannte Methode zur Herstellung von Alkyl-cyano-nitro-benzolen, besteht in dem bekannten Austausch der Aminogruppe von Alkyl-amino-nitrobenzolen gegen eine Nitrilgruppe, entsprechend J. Org. Chemistry 44, 4003 (1979).

Die bei der Nitrierung anfallenden 1-Alkyl-cyano-nitro-benzole oder deren Isomerengemische können direkt, d.h. ohne weitere Reinigung, z.B. analog dem in Farmaco (Pavia), Ediz. Sci. 25, 163 (1970) (C.A. 72, 121, 101d, 1970) für 2-Cyano-nitro-toluol beschriebenen Verfahren, zu 1-Alkyl-amino-methyl-amino-benzolen oder deren Isomerengemische reduziert werden. Die Reduktion der Nitro- und der Cyanogruppe kann hierbei in einem oder in zwei aufeinanderfolgenden Reaktionsschritten durchgeführt werden. Der Zusatz von Ammoniak ist nicht immer erforderlich.

Die Kernreduktion der 1-Alkyl-2-aminomethyl-amino-benzole (XI) bzw. der 1-Alkyl-4-aminomethyl-amino-benzole erfolgt nach bekannten Verfahren, z.B. nach den in Houben-Weyl, Methoden der organischen Chemie, 4. Auflage Band XI/1, Seiten 678 ff (Georg Thieme Verlag, Stuttgart 1957); C. Ferry, Reaktionen der organischen Synthese, Georg Thieme Verlag, Stuttgart 1978, Seiten 83, 90 ff; A.E. Barkdoll, A. al. J. Amer. Chem. Sol. 75, 1156 (1953), Chemistry Letters 1982, Seiten 603 bis 606, US-PA 2 494 563 oder DE-A 2 132 547 beschriebenen Verfahren.

Zur Darstellung der 1-Alkyl-2-aminomethyl-aminocyclohexane (V) bzw. der 1-Alkyl-4-amino-methyl-aminocyclohexane kann man auch unmittelbar von den 1-Alkyl-2-cyano-nitrobenzolen (X) ausgehen.

Die erhaltenen 1-Alkyl-aminomethyl-amino-cyclohexane können direkt oder als Salze, vorzugsweise Hydrochloride, in Lösungsmitteln phosgeniert werden. Geeignete Lösungsmittel sind beispielsweise Toluol, Xylol, Chlorbenzol oder Dichlorbenzol. Eine Lösung der 1-Alkyl-aminomethyl-amino-cyclohexane oder eine Suspension der entsprechenden Salze wird danach bei Temperaturen von ungefähr 0 °C bis 100 °C, vorzugsweise 10 °C bis 50 °C mit 1 bis 6 Mol, vorzugsweise 1 bis 2,5 Mol Phosgen pro $NH_2$- oder $NH_2 \cdot HCl$-Gruppen zur Reaktion gebracht und das intermediär gebildete Carbaminsäurechlorid bei Temperaturen von 80 °C bis 180 °C, vorzugsweise 120 °C bis 160 °C in das 1-Alkyl-isocyanatomethylisocyanatocyclohexan gespalten. Das gasförmige oder flüssige Phosgen wird hierbei der Reaktionsmischung mit einer solchen Geschwindigkeit zugeführt, daß die austretenden Gase überwiegend aus Chlorwasserstoff bestehen.

Nach beendeter Phosgenierung und Spaltung wird das Lösungsmittel, vorzugsweise unter vermindertem Druck, beispielsweise von 100 bis 10 mbar abdestilliert. Gegebenenfalls kann es auch vorteilhaft sein, den Chlorwasserstoff und evtl. vorliegendes überschüssiges Phosgen mit Hilfe von Stickstoff oder einem anderen Inertgas aus der Diisocyanatlösung auszutreiben, bevor das Lösungsmittel abdestilliert wird.

Die erhaltenen rohen 1-Alkyl-2- bzw. -4-isocyanatomethyl-isocyanato-cyclohexane oder Isomerengemische können durch Destillation unter vermindertem Druck getrennt und gereinigt werden.

Die erfindungsgemäßen Diisocyanate können ferner durch thermische Spaltung der entsprechenden Diurethane in der Gas- oder Flüssigphase, gegebenenfalls in Gegenwart von Katalysatoren hergestellt werden, wobei die Diurethane zweckmäßigerweise nach dem Verfahren der EP-OS 18 583 (US 4 278 805) durch Umsetzung von Carbaminsäureester mit den 1-Alkyl-aminocyclohexanen in Gegenwart von Alkoholen und gegebenenfalls Harnstoff erhalten werden.

Die neuen 1-Alkyl-2- bzw. -4-aminomethyl-aminocyclohexane und 1-Alkyl-2- bzw. -4-isocyanatomethyl-isocyanato-cyclohexane sind wertvolle Zwischenprodukte und Ausgangsmaterialien für Pflanzenschutzmittel und Kunststoffe.

Die Diamine werden vorzugsweise in Diisocyanate übergeführt. Diese eignen sich insbesondere zur Herstellung von Polyurethan-Schaumstoffen, -Klebstoffen, -Lacken, -Beschichtungsmitteln und -Dichtungsmitteln.

Die folgenden Beispiele sollen die Erfindung näher erläutern, ohne sie jedoch zu beschränken.

Beispiel 1

a) Herstellung eines Isomerengemisches aus 1-Methyl-2-cyano-4-nitrobenzol und 1-Methyl-2-cyano-6-nitrobenzol

In einem Reaktionsgefäß, ausgestattet mit Tropftrichter, Rührer und Thermometer, wurden 1200 Gewichtsteile konz. Schwefelsäure auf −5 °C abgekühlt. Unter gutem Rühren ließ man hierzu innerhalb von einer Stunde 200 Gewichtsteile 2-Cyano-toluol und danach zwischen −5 °C und +5 °C 151 Gewichtsteile konzentrierte Salpetersäure hinzutropfen. Zur Vervollständigung der Nitrierung rührte man noch 1,5 Stunden bei 0 °C und goß das Reaktionsgemisch nun auf 2000 Gewichtsteile Eis. Man saugte den Niederschlag ab, wusch das Filtergut gründlich mit Wasser und trocknete auf Ton.

Man erhielt 282 Gewichtsteile eines rohen Isomerengemisches aus 1-Methyl-2-cyano-4- und -6-nitrobenzol im Gewichtsverhältnis von ungefähr 87:13 mit einem Schmelzpunkt von 91 bis 92 °C.

b) Herstellung einer Isomerenmischung aus 1-Methyl-2-aminomethyl-4- und -6-amino-benzol

100 Gewichtsteile des gemäß (a) erhaltenen 1-Methyl-2-cyano-4- und -6-nitrobenzol-Isomerengemisches wurden in 800 Gewichtsteilen Ethanol gelöst und nach Zugabe von 25 Gewichtsteilen Raney-Nickel bei 50 bar und 80 °C hydriert. Nach dem Abkühlen auf Raumtemperatur wurde der Katalysator abfiltriert, das Filtrat unter vermindertem Druck eingeengt und der Rückstand destilliert. Man erhielt 76 Gewichtsteile eines bei 135 bis 140 °C (0,3 mbar) siedenden Gemisches aus 1-Methyl-2-aminomethyl-4- und -6-aminobenzol in Form eines Öls, das rasch erstarrte.

$C_8H_{12}N_2$ (Molekulargewicht 136, massenspektrometrisch).

c) Herstellung einer Isomerenmischung aus 1-Methyl-2-aminomethyl-4- und -6-amino-cyclohexan

50 Gewichtsteile des gemäß (b) erhaltenen 1-Methyl-2-aminomethyl-4- und -6-aminobenzols wurden in 400 Volumenteilen Dioxan in Gegenwart von 1,5 Gewichtsteilen Rutheniumoxidhydrat (hergestellt nach DE-PS 2 132 547) bei 150 °C und 250 bar hydriert. Nach dem Abkühlen auf Raumtemperatur wurde der Katalysator abfiltriert, das Filtrat unter vermindertem Druck eingeengt und der Rückstand destilliert. Man erhielt 32 Gewichtsteile eines bei 83 bis 85 °C (0,4 mbar) siedenden Gemisches aus 1-Methyl-2-aminomethyl-4- und -6-amino-cyclohexan.

Analyse: $C_8H_{18}N_2$ (Molekulargewicht: 142, massenspektrometrisch)

| | C | H | N |
|---|---|---|---|
| ber. | 67,55 | 12,75 | 19,69 |
| gef. | 67,30 | 12,70 | 19,60 |

d) Herstellung einer Isomerenmischung aus 1-Methyl-2-isocyanatomethyl-4- und -6-isocyanato-cyclohexan

Zu einer auf 0 °C abgekühlten Mischung aus 260 Gewichtsteilen o-Dichlorbenzol und 80 Gewichtsteilen Phosgen ließ man unter Rühren eine Lösung von 14,2 Gewichtsteilen der gemäß (c) erhaltenen Isomerenmischung aus 1-Methyl-2-aminomethyl-4- und -6-amino-cyclohexan in 100 Gewichtsteilen o-Dichlorbenzol hinzutropfen. Nach beendeter Zugabe wurde langsam auf 130 °C erwärmt und bei dieser Temperatur 1,5 Stunden lang Phosgen durch das Reaktionsgemisch geleitet. Danach ließ man das Reaktionsgemisch auf Raumtemperatur abkühlen und trennte das überschüssige Phosgen durch Einleiten von Stickstoff ab. Danach wurde zunächst das Lösungsmittel bei 10 bis 20 mbar abdestilliert und darauf der Rückstand im Hochvakuum destilliert. Man erhielt 10,5 g Gewichtsteile einer Mischung aus 1-Methyl-2-isocyanatomethyl-4- und -6-isocyanato-cyclohexan mit einem Siedepunkt von 110 bis 112 °C (ca. 0,1 mbar).

Analyse: $C_{10}H_{14}N_2O_2$ (Molekulargewicht 194, massenspektrometrisch)

| | C | H | N | O |
|---|---|---|---|---|
| ber. | 61,83 | 7,26 | 14,42 | 16,47 |
| gef. | 62,20 | 7,50 | 14,40 | 16,00 |

Beispiel 2

a) Herstellung von 1-Methyl-4-cyano-2-nitrobenzol

In einem Reaktionsgefäß, ausgestattet mit Tropftrichter, Rührer und Thermometer, wurden 240 Gewichtsteile konz. Schwefelsäure auf −5 °C gekühlt. Unter gutem Rühren trug man zunächst portionsweise 40 Gewichtsteile 4-Cyanotoluol ein und ließ danach zwischen −5 °C und +5 °C 25 Volumenteile konzentrierte Salpetersäure hinzutropfen. Zur Vervollständigung der Nitrierung rührte man noch 2 Stunden bei 0 °C und goß das Reaktionsgemisch nun auf 400 Gewichtsteile Eis. Man saugte den Niederschlag ab, wusch das Filtergut gründlich mit Wasser und trocknete an der Luft.

Man erhielt 50,9 Gewichtsteile 1-Methyl-4-cyano-2-nitrobenzol mit einem Schmelzpunkt von 105 bis 106 °C.

b) Herstellung von 1-Methyl-4-aminomethyl-2-aminobenzol

60 Gewichtsteile des gemäß a) erhaltenen 1-Methyl-4-cyano-2-nitro-benzols wurden in 800 Gewichtsteilen Ethanol gelöst und nach Zugabe von 20 Gewichtsteilen Raney-Nickel bei 10 bar und 60 °C hydriert bis die Wasserstoffaufnahme beendet war. Nach Zugabe von 8 Volumenteilen 3%iger Ammoniaklösung wurde erneut bei 10 bar und 60 °C hydriert. Nach dem Abkühlen auf Raumtemperatur wurde der Katalysator abfiltriert und das Filtrat unter vermindertem Druck eingeengt. Der Rückstand wurde im Hochvakuum destilliert. Man erhielt 32,1 Gewichtsteile 1-Methyl-4-aminomethyl-2-aminobenzol mit einem Siedepunkt von 130 bis 135 °C (0,3 mbar).

| | C | H | N |
|---|---|---|---|
| ber. | 70,55 | 8,88 | 20,57 |
| gef. | 70,80 | 9,00 | 19,90 |

c) Herstellung von 1-Methyl-4-aminomethyl-2-amino-cyclohexan

20 Gewichtsteile des gemäß (b) erhaltenen 1-Methyl-4-aminomethyl-2-aminobenzols wurden in 150 Volumenteilen Dioxan in Gegenwart von 0,5 Gewichtsteilen Rutheniumoxidhydrat (hergestellt nach DE-PS 2 132 547) bei 150 °C und 250 bar hydriert. Nach dem Abkühlen auf Raumtemperatur wurde der Katalysator abfiltriert, das Filtrat unter vermindertem Druck eingeengt und der Rückstand destilliert. Man erhielt 14,4 Gewichtsteile 1-Methyl-4-aminomethyl-2-aminocyclohexan vom Siedepunkt 60 bis 63 °C (0,04 mbar).

Analyse: $C_8H_{18}N_2$ (Molekulargewicht 142, massenspektrometrisch)

| | C | H | N |
|---|---|---|---|
| ber. | 67,55 | 12,75 | 19,69 |
| gef. | 67,40 | 12,60 | 19,90 |

d) Herstellung von 1-Methyl-4-isocyanatomethyl-2-isocyanatocyclohexan

Zu einer auf 0 °C abgekühlten Mischung aus 150 Gewichtsteilen o-Dichlorbenzol und 55 Gewichtsteilen Phosgen ließ man unter Rühren eine Lösung aus 9,1 Gewichtsteilen des gemäß c) erhaltenen 1-Methyl-4-aminomethyl-2-aminocyclohexans in 75 Gewichtsteilen o-Dichlorbenzol tropfen. Nach beendeter Zugabe wurde langsam auf 130 °C erwärmt und bei dieser Temperatur 15 Stunden lang Phosgen durch das Reaktionsgemisch geleitet.

Danach ließ man das Reaktionsgemisch auf Raumtemperatur abkühlen und trennte das überschüssige Phosgen durch Einleiten von Stickstoff

ab. Danach wurde zunächst das Lösungsmittel bei 10 bis 20 mbar abdestilliert und darauf der Rückstand im Hochvakuum destilliert. Man erhielt 2,4 Gewichtsteile 1-Methyl-4-isocyanatomethyl-2-isocyanatocyclohexan mit einem Siedepunkt von 98 bis 102 °C (0,4 mbar).

Analyse: $C_{10}H_{14}N_2O_2$ (Molekulargewicht 194, massenspektrometrisch).

**Patentansprüche**

1. Diisocyanate der Formeln

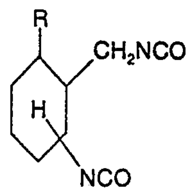
(I) oder
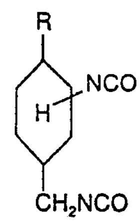
(II),

in denen R eine Alkylgruppe mit 1 bis 12 Kohlenstoffatomen bedeutet.

2. Diisocyanate der Formeln

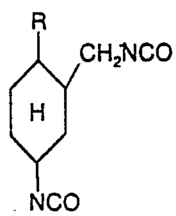
(III) oder
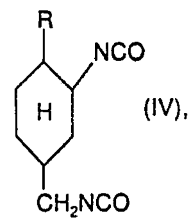
(IV),

in denen R eine Alkylgruppe mit 1 bis 12 Kohlenstoffatomen bedeutet.

3. Mischungen aus Diisocyanaten der Formeln (I) und/oder (II).

4. Mischungen aus Diisocyanaten der Formeln (III) und/oder (IV).

5. Diisocyanate der Formeln (I), (II), (III) oder (IV), in denen R eine Methyl-, Ethyl-, n-Propyl-, iso-Propyl- oder n-Butylgruppe ist.

6. Mischung aus 1-Methyl-2-isocyanatomethyl-4-isocyanatocyclohexan und 1-Methyl-2-isocyanatomethyl-6-isocyanato-cyclohexan.

7. 1-Methyl-2-isocyanatomethyl-4-isocyanato-cyclohexan.

8. 1-Methyl-2-isocyanatomethyl-6-isocyanato-cyclohexan.

9. 1-Methyl-4-isocyanatomethyl-2-isocyanato-cyclohexan.

10. Diamine der Formeln

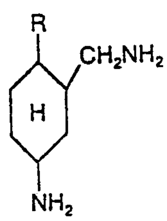
(VII) oder
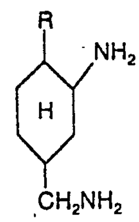
(VIII)

in denen R eine Alkylgruppe mit 1 bis 12 Kohlenstoffatomen bedeutet, sowie Mischungen aus Diaminen der Formeln (VII) und/oder (VIII).

11. Diamine der Formeln (VII) oder (VIII), in denen R eine Methyl-, Ethyl-, n-Propyl-, iso-Propyl- oder n-Butylgruppe ist.

12. Mischung aus
1-Methyl-2-aminomethyl-4-aminocyclohexan und
1-Methyl-2-aminomethyl-6-aminocyclohexan.

13. 1-Methyl-2-aminomethyl-4-aminocyclohexan.

14. 1-Methyl-2-aminomethyl-6-aminocyclohexan.

15. 1-Methyl-4-aminomethyl-2-aminocyclohexan.

16. Verfahren zur Herstellung von Diisocyanaten der Formeln (I) und (II), dadurch gekennzeichnet, daß man Diamine der Formeln

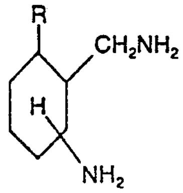
(V) oder
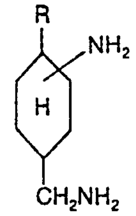
(VI),

phosgeniert und die hierbei entstehenden Carbaminsäurechloride thermisch spaltet.

17. Verwendung von Diisocyanaten der Formeln (I) und/oder (II) zur Herstellung von Polyurethan-Kunststoffen oder Pflanzenschutzmitteln.

## Claims

1. A diisocyanate of the formula

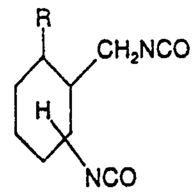 (I) or 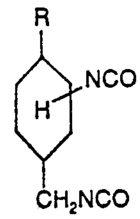 (II),

where R is alkyl of from 1 to 12 carbon atoms.

2. A diisocyanate of the formula

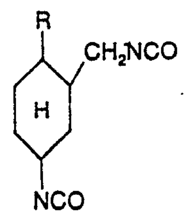 (III) or 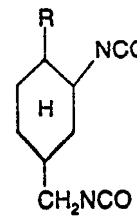 (IV),

where R is alkyl of from 1 to 12 carbon atoms.

3. A mixture of diisocyanates of the formulae (I) and/or (II).

4. A mixture of diisocyanates of the formulae (III) and/or (IV).

5. A diisocyanate of the formula (I), (II), (III) or (IV) where R is methyl, ethyl, n-propyl, isopropyl or n-butyl.

6. A mixture of 1-methyl-2-isocyanatomethyl-4-isocyanatocyclohexane and 1-methyl-2-isocyanatomethyl-6-isocyanatocyclohexane.

7. 1-Methyl-2-isocyanatomethyl-4-isocyanatocyclohexane.

8. 1-Methyl-2-isocyanatomethyl-6-isocyanatocyclohexane.

9. 1-Methyl-4-isocyanatomethyl-2-isocyanatocyclohexane.

10. A diamine of the formula

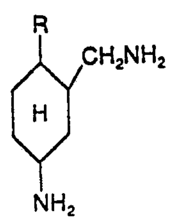 (VII) or 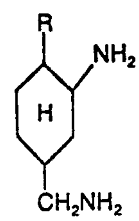 (VIII)

where R is alkyl of from 1 to 12 carbon atoms, or a mixture of diamines of the formulae (VII) and/or (VIII).

11. A diamine of the formula (VII) or (VIII) where R is methyl, ethyl, n-propyl, isopropyl or n-butyl.

12. A mixture of 1-methyl-2-aminomethyl-4-aminocyclohexane and 1-methyl-2-aminomethyl-6-aminocyclohexane.

13. 1-Methyl-2-aminomethyl-4-aminocyclohexane.

14. 1-Methyl-2-aminomethyl-6-aminocyclohexane.

15. 1-Methyl-4-aminomethyl-2-aminocyclohexane.

16. A process for preparing a diisocyanate of the formula (I) or (II), which comprises phosgenating a diamine of the formula

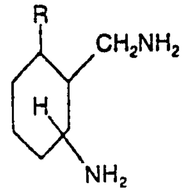 (V) or 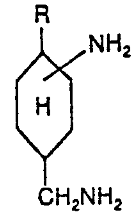 (VI),

and thermally cleaving the resulting carbamoyl chloride.

17. The use of a diisocyanate of the formula (I) or (II) for preparing polyurethane plastics or crop protection agents.

**Revendications**

1. Diisocyanates de l'une des formules

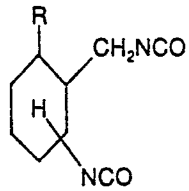

(I) et

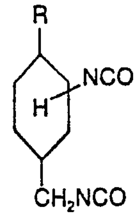

(II),

dans lesquelles R représente un radical alkyle en $C_1$ à $C_{12}$.

2. Diisocyanates de l'une des formules

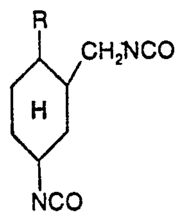

(III) et

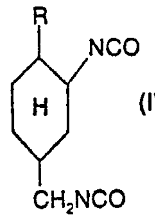

(IV),

dans lesquelles R représente un radical alkyle en $C_1$ à $C_{12}$.

3. Mélanges de diisocyanates des formules (I) et/ou (II).

4. Mélanges de diisocyanates des formules (III) et/ou (IV).

5. Diisocyanates des formules (I), (II), (III) ou (IV), pour lesquels R désigne un radical méthyle, éthyle, n-propyle, iso-propyle ou n-butyle.

6. Mélange de 1-méthyl-2-isocyanatométhyl-4-isocyanato-cyclohexane et de 1-méthyl-2-isocyanatométhyl-6-isocyanato-cyclohexane.

7. Le 1-méthyl-2-isocyanatométhyl-4-isocyanato-cyclohexane.

8. Le 1-méthyl-2-isocyanatométhyl-6-isocyanato-cyclohexane.

9. Le 1-méthyl-4-isocyanatométhyl-2-isocyanato-cyclohexane.

10. Diamines de l'une des formules

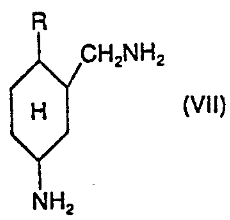

(VII) et

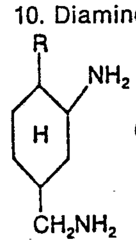

(VIII)

dans lesquelles R représente un radical alkyle en $C_1$ à $C_{12}$, ainsi que les mélanges de diamines des formules (VII) et/ou (VIII).

11. Diamines des formules (VII) et/ou (VIII), pour lesquelles R désigne un radical méthyle, éthyle, n-propyle, iso-propyle ou n-butyle.

12. Mélange de 1-méthyl-2-aminométhyl-4-amino-cyclohexane et de 1-méthyl-2-aminométhyl-6-amino-cyclohexane.

13. Le 1-méthyl-2-aminométhyl-4-amino-cyclohexane.

14. Le 1-méthyl-2-aminométhyl-6-amino-cyclohexane.

15. Le 1-méthyl-4-aminométhyl-2-amino-cyclohexane.

16. Procédé de préparation de diisocyanates des formules (I) et (II), caractérisé en ce que l'on soumet des diamines des formules

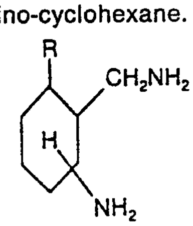

(V) et

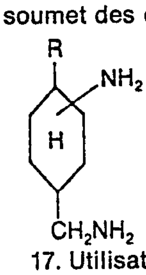

(VI),

à une phosgénation et les chlorures d'acide carbamique, qui se forment, à une décomposition thermique.

17. Utilisation de diisocyanates des formules (I) et/ou (II) pour la fabrication de matières plastiques polyuréthanniques et/ou de substances phytosanitaires.